(19) Europäisches Patentamt
European Patent Office
Office européen des brevets

(11) Veröffentlichungsnummer: **0 264 089 B1**

(12) **EUROPÄISCHE PATENTSCHRIFT**

(45) Veröffentlichungstag der Patentschrift: **08.07.92**

(51) Int. Cl.$^5$: **C07D 213/20**, C07D 215/10, C07D 233/60

(21) Anmeldenummer: **87114833.4**

(22) Anmeldetag: **10.10.87**

# (54) Verfahren zur Herstellung von Sulfatobetainen.

(30) Priorität: **16.10.86 DE 3635230**

(43) Veröffentlichungstag der Anmeldung:
**20.04.88 Patentblatt 88/16**

(45) Bekanntmachung des Hinweises auf die Patenterteilung:
**08.07.92 Patentblatt 92/28**

(84) Benannte Vertragsstaaten:
**CH DE FR GB IT LI**

(56) Entgegenhaltungen:
**DE-A- 1 906 851**
**US-A- 3 131 189**
**US-A- 3 178 446**
**US-A- 3 274 204**
**US-A- 3 314 868**

(73) Patentinhaber: **BASF Aktiengesellschaft**
**Carl-Bosch-Strasse 38**
**W-6700 Ludwigshafen(DE)**

(72) Erfinder: **Braun, Gerold, Dr.**
**Limesstrasse 3**
**W-6700 Ludwigshafen(DE)**
Erfinder: **Tschang, Chung-Ji, Dr.**
**Hinterbergstrasse 31**
**W-6702 Bad Dürkheim(DE)**
Erfinder: **Vamvakaris, Christos, Dr.**
**Riedweg 6**
**W-6701 Kallstadt(DE)**
Erfinder: **Glaser, Klaus**
**Neuweg 14**
**W-6704 Mutterstadt(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Herstellung von Sulfatobetainen durch Umsetzung von Additionsverbindungen aus einer Base mit einem tertiären N-Atom und Schwefeltrioxid mit einem Alkylencarbonat.

In der US-PS 3 274 204 wird die Herstellung von Sulfatobetainen durch Umsetzung eines Epoxids mit einem Komplex aus einem tertiären Amin und Schwefeltrioxid, durch Umsetzung eines cyclischen Sulfats mit einem tertiären Amin oder durch Umsetzung eines Epoxids mit dem Komplex aus Dioxan und Schwefeltrioxid und anschließender Umsetzung mit einem tertiären Amin beschrieben.

Diese Verfahren werden zweckmäßigerweise, wie aus der Beschreibung und den Ausführungsbeispielen hervorgeht, in Gegenwart eines inerten Lösungsmittels, wie Toluol, Hexan, Ethylendichlorid, die unter Umständen toxikologisch nicht ganz unbedenklich sind, durchgeführt. Falls von einem cyclischen Sulfat als Ausgangsmaterial ausgegangen wird, handelt es sich um Verbindungen, die an sich nicht leicht zugänglich sind. Im übrigen hat sich gezeigt, daß die beschriebenen Verfahren im Hinblick auf Ausbeute und Reinheit der erhaltenen Produkte nicht immer befriedigen. Bei der Verwendung von leichtflüchtigen Epoxiden sind zusätzlich aufwendige Sicherheitsvorkehrungen erforderlich. Das trifft in gleicher Weise auch für das in der DE-OS 19 06 851 beschriebene Verfahren zu.

Aus der DE-AS 11 91 652 geht hervor, daß beispielsweise Pyridiniumethylsulfat (2-Pyridinium-1-sulfatoethan) in aufwendiger Weise durch Umsetzung von Hydroxiethylpyridiniumchlorid, erhalten aus Pyridin und Ethylenchlorhydrin, mit Chlorsulfonsäure hergestellt werden kann. Auch bei dieser Arbeitsweise lassen Ausbeute und Reinheit zu wünschen übrig.

Es bestand daher die Aufgabe, ein großtechnisch möglichst einfach durchführbares Verfahren zur herstellung von Sulfatobetainen in hoher Reinheit und Ausbeuten zur Verfügung zu stellen.

Gegenstand der Erfindung ist ein Verfahren zur Herstellung von Sulfatobetainen der Formel I

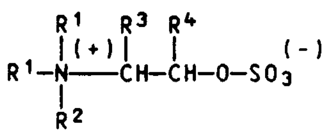

(I),

in der die einzelnen Reste $R^1$ und $R^2$ gleich oder verschieden sein können und einen gesättigten geradkettigen oder verzweigten Alkylrest mit 1 bis 22 C-Atomen, insbesondere 1 bis 7 C-Atomen, einen Cycloalkylrest mit 5 bis 7 C-Atomen im Ring, einen Phenyl- oder Naphthylrest oder einen Aralkylrest mit insgesamt 7 bis 12 C-Atomen und darüber hinaus die beiden Reste $R^1$ zusammen mit dem N-Atom einen 5- oder 6-gliedrigen heterocyclischen Ring, der gegebenenfalls ein oder mehrere weitere Heteroatome und gegebenenfalls einen annelierten Benzolring enthält und wobei für $R^2$ ein niederer Alkylrest mit 1 bis 4 C-Atomen steht, oder die beiden Reste $R^1$ zusammen mit dem N-Atom und mit dem Rest $R^2$ einen 5- oder 6-gliedrigen ungesättigten heterocyclischen Ring, der gegebenenfalls ein oder mehrere weitere Heteroatome und gegebenenfalls einen annelierten Benzolring enthält, und $R^3$ und $R^4$ gleich oder verschieden sind und $R^3$ ein Wasserstoffatom oder einen Alkylrest mit 1 bis 7 C-Atomen und $R^4$ ein Wasserstoffatom, einen Alkylrest mit 1 bis 20 C-Atomen oder Phenyl, wobei $R^3$ und $R^4$ bevorzugt Wasserstoff oder Wasserstoff und Methyl und beide Reste miteinander vertauscht sein können, bedeuten, aus Additionsverbindungen von Basen mit einem tertiären N-Atom und Schwefeltrioxid,
das dadurch gekennzeichnet ist, daß man ein Amin-$SO_3$-Addukt der Formel II

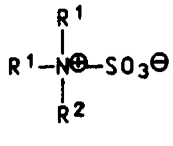

(II),

in der $R^1$ und $R^2$ die für Formel I angegebenen Bedeutungen aufweisen, mit einem Alkylencarbonat der Formel III

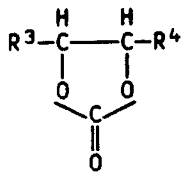

(III),

in der $R^3$ und $R^4$ die für Formel I angegebenen Bedeutungen aufweisen, bei Temperaturen von 80 bis 220°C, bevorzugt 100 bis 160°C, in Gegenwart einer überschüssigen Menge an Alkylencarbonat der Formel III als Lösungsmittel und/oder einer überschüssigen Menge der dem $SO_3$-Addukt der Formel II zugrundeliegenden Base als Lösungsmittel und/oder eines inerten Lösungsmittels umsetzt.

Das erfindungsgemäße Verfahren liefert in überraschender Einfachheit in üblichen Rührkesseln unter Vermeidung aufwendiger Apparaturen und weitgehender Verminderung des Umgangs mit hochtoxischen Verbindungen und Lösungsmitteln Sulfatobetaine in hoher Reinheit und hohen Ausbeuten über 90 %.

Im einzelnen wird hierzu erläutert: Hervorzuhebende tertiäre Amine als Ausgangsverbindungen für die Amin-$SO_3$-Additionsverbindungen der Formel II sind Tridodecyl-, Tristearyl-, Tricyclohexyl-, Triphenyl-, Dimethyl-dodecyl-, Diethylphenyl-, Dimethyl-stearyl-, Trimethyl-, Triethyl- oder Tributylamin. Zu den bevorzugten tertiären Alkylaminen gehören tertiäre Amine mit Alkylresten mit 1 bis 4 C-Atomen für jeweils $R_1$ und 12 bis 18 C-Atomen für $R^2$.

Besonders bevorzugt sind Additionsverbindungen des Schwefeltrioxids an heterocyclishe Verbindungen. Hervorzuhebende heterocyclische Verbindungen sind beispielsweise N-Methyl- und N-Ethylpiperidin, N-Methyl- und N-Ethylmorpholin, N-Methylpyrazol, Oxazol, Isoxazol, Acridin, Phenacridin, Pyrazin und Pyridazin.

Besonders bevorzugt sind Pyridin, ggfs. durch ein oder zwei Alkylreste mit 1 bis 4 C-Atomen, eine Carboxylalkylgruppe mit 1 bis 4 C-Atomen im Alkyl oder eine Nitrilgruppe substituiert, wie $\alpha$, $\beta$-, $\gamma$-Picolin, 2-Ethylpyridin, Nicotinsäuremethylester oder Nicotinsäurenitril, Chinolin oder Isochinolin, ggfs. durch einen Methylrest substituiert, wie Chinaldin, und N-($C_1$- bis $C_{12}$-Alkyl)-imidazole, ggfs. an einem der C-Atome durch einen Alkylrest mit 1 bis 6 C-Atomen oder Phenyl substituiert, wie N-Methyl- und N-Ethylimidazol, 1,2-Dimethylimidazol, N-Methyl-2-Phenylimidazol oder 1-Dodecylimidazol.

Die Herstellung der $SO_3$-Additionsverbindungen der Formel II kann in bekannter und üblicher Weise erfolgen oder im Reaktionsgemisch, wie aus den Beispielen hervorgeht.

Die 1,2-Alkylencarbonate der Formel III sind grundsätzlich bekannt und können beispielsweise aus dem entsprechenden Epoxid und Kohlendioxid unter Druck hergestellt werden. Es wird darauf hingewisen, daß Ausgangsverbindungen mit mehreren cyclischen Carbonatgruppen Di- und Polysulfatobetaine ergeben.

Ganz besonders bevorzugt als Ausgangsverbindungen der Formel III sind das Ethylencarbonat und das 1,2-Proplyencarbonat.

Wie oben erwähnt, erfolgt die Umsetzung einer Verbindung der Formel II mit einem Alkylencarbonat der Formel III bei Temperaturen von 80 bis 220°C, bevorzugt 100 bis 160°C. Die Umsetzung erfolgt in Molverhältnissen $SO_3$-Additionsprodukt : Alkylencarbonat von 1:1 bis 1:5. Bevorzugt sind Molverhältnisse von 1:2,5 bis 1:4, wobei das überschüssige cyclische Carbonat als zweckmäßiges Lösungsmittel dient.

Es ist auch möglich, insbesondere wenn bei Molverhältnissen von 1:1 gearbeitet wird, die der $SO_3$-Additionsverbindung zugrundeliegende Base in überschüssiger Menge als Lösungsmittel zu verwenden.

Überschüssige Base führt zu einer beschleunigten Umsetzung. Wegen dieses Effektes empfiehlt es sich, die erfindungsgemäße Reaktion unabhängig vom verwendeten Lösungsmittel in Gegenwart einer katalytischen Menge der der $SO_3$-Additionsverbindung der Formel II zugrundeliegenden Base durchzuführen. Vorteilhaft sind, bezogen auf das $SO_3$-Addukt, Mengen von 0,01 bis 10 Mol%, bevorzugt 0,1 bis 5,0 Mol%.

Weiterhin können inerte Lösungsmittel, insbesondere wenn feste cyclische Carbonate der Formel III vorliegen, verwendet werden. Zweckmäßig sind dipolare aprotische Lösungsmittel, wie Dimethylformamid, Dimethylpropylenharnstoff oder Sulfolan. Selbstverständlich können auch Mischungen der verwendeten Lösungsmittel verwendet werden.

Die erfindungsgemäße Umsetzung kann für den Fall der Verwendung der Additionsverbindung von Schwefeltrioxid an Pyridin und Umsetzung mit Ethylencarbonat durch folgende Formeln wiedergegeben werden:

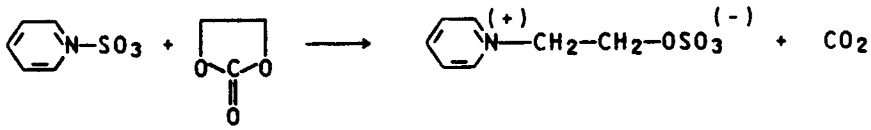

Zweckmäßigerweise wird zunächst die $SO_3$-Additionsverbindung in überschüssiger Menge Base, in Gegenwart eines Lösungsmittels oder in Gegenwart gegebenenfalls überschüssigen Alkylencarbonats bei Temperaturen von 40 bis 65 °C hergestellt. Anschließend wird durch Erhöhung der Temperatur auf wenigstens 80 °C die eigentliche Umsetzung zum Betain durchgeführt.

Die vollständige Umsetzung ist erreicht, wenn kein $SO_3$-Additionsprodukt mehr nachweisbar ist. Zweckmäßig verfährt man so, daß kurz vor dem völligen Verschwinden der $SO_3$-Additionsverbindung die Umsetzung durch Kühlung beendet wird. Dadurch werden Verfärbungen der Lösung vermieden. Im übrigen kann die erfindungsgemäße Reaktion kontinuierlich oder diskontinuierlich durchgeführt werden.

Die häufig kristallin anfallenden Betaine können in einfacher Weise durch Absaugen abgetrennt werden. Nach dem Waschen mit einem Lösungsmittel, wie Ethanol oder Methanol, werden praktisch analysenreine Produkte erhalten.

Die nach dem erfindungsgemäßen Verfahren hergestellten Verbindungen können als Tenside, insbesondere in Waschmitteln,oder als Hilfsmittel auf dem Textilgebiet oder als Galvanisierhilfsmittel sowie als Netzmittel, Emulgatoren oder Dispergatoren verwendet werden.

Durch das erfindungsgemäße Verfahren werden insbesondere heterocyclische Ethanosulfate, die als Galvanisierhilfsmittel hervorragende Spitzenglanzbildner sind, leicht zugänglich. Hiervon sind beispielsweise zu nennen das Pyridiniumsulfatoethan oder das Chinoliniumsulfatoethan.

Beispiel 1

In einem Rührkessel werden 261 Teile Pyridin und 792 Teile Ethylencarbonat vorgelegt. Nach der Zugabe von 240 Teilen $SO_3$ bei 60 °C zur Herstellung der Additionsverbindung wird die Temperatur auf 130 °C erhöht. Gegen Ende der Reaktion scheidet sich das Pyridiniumsulfatoethan kristallin ab. Die Suspension wird abgekühlt, die Kristalle werden abgetrennt und mit Methanol gewaschen. Schmelzpunkt: 203 - 205 °C, Ausbeute: 92 % der Theorie, bezogen auf eingesetztes $SO_3$.

Die Analyse der angefallenen Kristalle ohne weitere Reinigung durch Kristallisation zeigt, daß ein analysenreines Produkt erhalten worden ist.

**Elementaranalyse:**
**Molgewicht: 203**

| | C | H | N | S |
|---|---|---|---|---|
| Ber. | 41.37 | 4.43 | 6.89 | 15.7 |
| Gef. | 41.3 | 4.5 | 6.9 | 15.5 |

Beispiel 2

Zur Herstellung des $SO_3$-Addukts werden 237 Teile Pyridin in einem Rührkessel vorgelegt und bei Temperaturen von 60 °C 80 Teile $SO_3$ eindosiert. Anschließend werden 88 Teile Ethylencarbonat zugetropft und unter Erhitzen auf Siedetemperaturen wird unter Rückfluß gerührt bis zur vollständigen Umsetzung. Nach dem Abkühlen wird abgesaugt und mit Methanol gewaschen. Es fallen analysenreine Kristalle mit einem Schmelzpunkt von 200 °C in einer Ausbeute von 90 % der Theorie, bezogen auf eingesetztes $SO_3$ an.

Beispiel 3

100 Teile Dimethylpropylenharnstoff als Lösungsmittel werden zusammen mit 40 Teilen Pyridin vorgelegt. Nach dem Eindosieren von 39 Teilen $SO_3$ bei 60 °C zur Herstellung der $SO_3$-Additionsverbindung werden 43 Teile Ethylencarbonat zugegeben. Die Reaktion wird bis zur vollständigen Umsetzung bei

140 ° C gerührt und dann unter 100 ° C gekühlt. Es wird abgesaugt und mit Methanol gewaschen. Die erhaltenen Kristalle vom Schmelzpunkt von 203 ° C sind analysenrein und es wird eine Ausbeute von 89 % der Theorie, bezogen auf eingesetztes $SO_3$, erhalten.

Entsprechend Beispiel 1 und in den dort verwendeten molaren Verhältnissen werden hergestellt:
Chinoliniumsulfatoethan vom Schmp. 305 ° C und mit 90 % Ausbeute,
N-Methylimidazoliumsulfatoethan vom Schmp. 196 ° C und mit 90 % Ausbeute,
Pyridiniumsulfatopropan,
1,2-Dimethylimidazoliumsulfatoethan und
3-Methylpyridiniumsulfatopropan.

## Patentansprüche

1. Verfahren zur Herstellung von Sulfatobetainen der Formel I

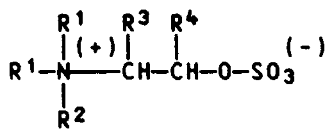

(I),

in der die einzelnen Reste $R^1$ und $R^2$ gleich oder verschieden sein können und einen gesättigten geradkettigen oder verzweigten Alkylrest mit 1 bis 22 C-Atomen, einen Cycloalkylrest mit 5 bis 7 C-Atomen im Ring, einen Phenyl- oder Naphthylrest oder einen Aralkylrest mit insgesamt 7 bis 12 C-Atomen und darüber hinaus die beiden Reste $R^1$ zusammen mit dem N-Atom einen 5- oder 6-gliedrigen heterocyclischen Ring, der gegebenenfalls ein oder mehrere weitere Heteroatome und gegebenenfalls einen annelierten Benzolring enthält und wobei für $R^2$ ein Alkylrest mit 1 bis 4 C-Atomen steht, oder die beiden Reste $R^1$ zusammen mit dem N-Atom und dem Rest $R^2$ einen 5- oder 6-gliedrigen ungesättigten heterocyclischen Ring, der gegebenenfalls ein oder mehrere weitere Heteroatome und gegebenenfalls einen annelierten Benzolring enthält, $R^3$ und $R^4$ gleich oder verschieden sind und $R^3$ ein Wasserstoffatom oder einen Alkylrest mit 1 bis 7 C-Atomen und $R^4$ ein Wasserstoffatom, einen Alkylrest mit 1 bis 20 C-Atomen oder einen Phenylrest, wobei die Reste $R^3$ und $R^4$ miteinander vertauscht sein können, bedeuten, aus Additionsverbindungen von Basen mit einem tertiären N-Atom und Schwefeltrioxid, dadurch gekennzeichnet, daß man ein Amin-$SO_3$-Addukt der Formel II

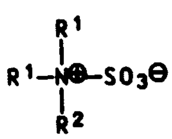

(II),

in der $R^1$ und $R^2$ die für Formel I angegebenen Bedeutungen aufweisen, mit einem Alkylencarbonat der Formel III

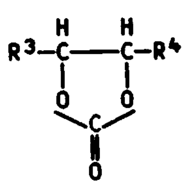

(III),

in der $R^3$ und $R^4$ die für Formel I angegebenen Bedeutungen aufweisen, bei Temperaturen von 80 bis 220 ° C in Gegenwart einer überschüssigen Menge an Alkylencarbonat als Lösungsmittel und/oder einer überschüssigen Menge der dem $SO_3$-Addukt zugrundeliegenden Base als Lösungsmittel und/oder eines inerten Lösungsmittels umsetzt.

2. Verfahren nach Anspruch 1, dadurch gekennzeichnet, daß man eine heterocyclische Additionsverbindung des Schwefeltrioxids an Pyridin, gegebenenfalls durch ein oder zwei Alkylreste mit 1 bis 4 C-Atomen, eine Carboxyalkylgruppe mit 1 bis 4 C-Atomen im Alkyl oder eine Nitrilgruppe substituiert, Chinolin oder Isochinolin, gegebenenfalls durch eine Methylgruppe substituiert, oder ein N-($C_1$- bis $C_{12}$-Alkyl)-imidazol, gegebenenfalls an einem der C-Atome durch einen Alkylrest mit 1 bis 6 C-Atomen oder Phenyl substituiert, einsetzt.

**3.** Verfahren nach den Ansprüchen 1 und/oder 2, dadurch gekennzeichnet, daß man als cyclisches Carbonat Ethylencarbonat oder 1,2-Propylencarbonat einsetzt.

**4.** Verfahren nach einem der Ansprüche 1 bis 3, dadurch gekennzeichnet, daß die Umsetzung in Gegenwart von 0,01 bis 10 Mol% des der $SO_3$-Additionsverbindung zugrundeliegenden Base, bezogen auf die $SO_3$-Additionsverbindung, durchgeführt wird.

**5.** Verfahren nach einem der Ansprüche 1 bis 4, dadurch gekennzeichnet, daß man die Additionsverbindung des Schwefeltrioxids an Pyridin mit Ethylencarbonat umsetzt.

## Claims

**1.** A process for the preparation of a sulfatobetaine of the formula I

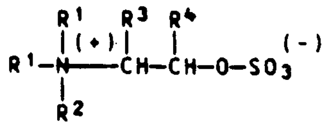

(I)

where the individual radicals $R^1$ and $R^2$ may be identical or different and are each a saturated straight-chain or branched alkyl radical of 1 to 22 carbon atoms, cycloalkyl where the ring is of 5 to 7 carbon atoms, phenyl or naphthyl, or aralkyl having a total of 7 to 12 carbon atoms, and moreover the two radicals $R^1$, together with the N atom, may form a 5-membered or 6-membered heterocyclic ring which may contain one or more further hetero atoms and may contain a fused benzene ring, and $R^2$ is alkyl of 1 to 4 carbon atoms, or the two radicals $R^1$, together with the N atom and $R^2$, form a 5-membered or 6-membered unsaturated heterocyclic ring which may contain one or more further hetero atoms and may contain a fused benzene ring, $R^3$ and $R^4$ are identical or different and $R^3$ is hydrogen or alkyl of 1 to 7 carbon atoms and $R^4$ is hydrogen, alkyl of 1 to 20 carbon atoms or phenyl, $R^3$ and $R^4$ being interchangeable, from an addition compound of a base having a tertiary N atom and sulfur trioxide, wherein an amine/$SO_3$ adduct of the formula II

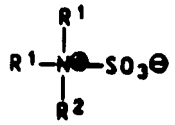

(II)

where $R^1$ and $R^2$ have the meanings stated for formula I, is reacted with an alkylene carbonate of the formula III

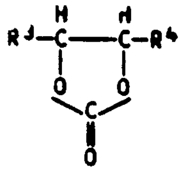

(III)

where $R^3$ and $R^4$ have the meanings stated for formula I, at from 80 to 220 °C in the presence of an excess of the alkylene carbonate as a solvent and/or an excess of the base used to prepare the $SO_3$ adduct, as a solvent, and/or an inert solvent.

**2.** A process as claimed in claim 1, wherein a heterocyclic addition compound of sulfur trioxide with

pyridine, which is unsubstituted or substituted by one or two alkyl radicals of 1 to 4 carbon atoms, by carboxyalkyl where alkyl is of 1 to 4 carbon atoms or by nitrile, with quinoline or isoquinoline, which is unsubstituted or substituted by methyl, or with an N-($C_1$-$C_{12}$-alkyl)-imidazole, which is unsubstituted or substituted at one of the carbon atoms by alkyl of 1 to 6 carbon atoms or by phenyl, is used.

**3.** A process as claimed in claims 1 and/or 2, wherein the cyclic carbonate used is ethylene carbonate or 1,2-propylene carbonate.

**4.** A process as claimed in any of claims 1 to 3, wherein the reaction is carried out in the presence of from 0.01 to 10 mol %, based on the $SO_3$ addition compound, of the base used to prepare the $SO_3$ addition compound.

**5.** A process as claimed in any of claims 1 to 4, wherein the addition compound of sulfur trioxide with pyridine is reacted with ethylene carbonate.

**Revendications**

**1.** Procédé de préparation de sulfatobétaïnes de formule I

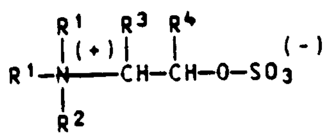

(I),

dans laquelle les restes $R^1$ et $R^2$ peuvent être identiques ou différents et représentent chacun un reste alkyle saturé à 1-22 atomes de carbone en chaîne droite ou ramifiée, un reste cycloalkyle à 5-7 atomes de carbone dans le cycle, un reste phényle ou naphtyle ou un reste aralkyle à 7-12 atomes de carbone au total, les deux restes $R^1$ pouvant en outre former, avec l'atome d'azote, un cycle penta- ou hexagonal hétérocyclique qui contient éventuellement un ou plusieurs autres hétéroatomes et éventuellement un noyau benzénique condensé, $R^2$ étant alors un reste alkyle à 1-4 atomes de carbone, ou les deux restes $R^1$ pouvant former, avec l'atome d'azote et le reste $R^2$, un noyau penta- ou hexagonal hétérocyclique insaturé qui contient éventuellement un ou plusieurs autres hétéroatomes et éventuellement un noyau benzénique condensé, $R^3$ et $R^4$ sont identiques ou différents, $R^3$ représentant un atome d'hydrogène ou un reste alkyle à 1-7 atomes de carbone et $R^4$ représentant un atome d'hydrogène, un reste alkyle à 1-20 atomes de carbone ou un reste phényle, les restes $R^3$ et $R^4$ pouvant être intervertis, à partir de composés d'addition de bases comportant un atome d'azote tertiaire et d'anhydride sulfurique, caractérisé en ce qu'on fait réagir un produit d'addition amine-$SO_3$ de formule II

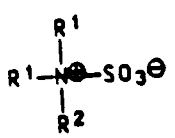

(II),

dans laquelle $R^1$ et $R^2$ ont les significations indiquées à propos de la formule I, avec un carbonate d'alkylène de formule III

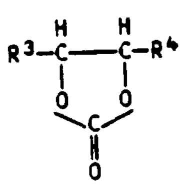

(III),

dans laquelle $R^3$ et $R^4$ ont les significations indiquées à propos de la formule I, à des températures de

80 à 220°C, en présence d'une quantité en excès de carbonate d'alkylène servant de solvant et/ou d'une quantité en excès de la base entrant dans la constitution du produit d'addition de $SO_3$ et servant de solvant et/ou d'un solvant inerte.

**2.** Procédé selon la revendication 1, caractérisé en ce qu'on utilise un compose d'addition hétérocyclique de l'anhydride sulfurique sur la pyridine, éventuellement substituée par un ou deux restes alkyle à 1-4 atomes de carbone, par un groupement carboxyalkyle à 1-4 atomes de carbone dans le radical alkyle ou par un groupement nitrile, sur la quinoléine ou la isoquinoléine, éventuellement substituée par un groupement méthyle, ou sur un N-(alkyl en $C_1$-$C_{12}$)imidazole, éventuellement substitué sur l'un des atomes de carbone par un reste alkyle à 1-6 atomes de carbone ou par un reste phényle.

**3.** Procédé selon la revendication 1 et/ou 2, caractérisé en ce qu on utilise, comme carbonate cyclique, du carbonate d'éthylène ou du carbonate de 1,2-propylène.

**4.** Procédé selon l'une quelconque des revendications 1 à 3, caractérisé en ce que la réaction est conduite en présence de 0,01 à 10% en moles de la base entrant dans la constitution du composé d'addition de $SO_3$, par rapport au composé d'addition de $SO_3$.

**5.** Procédé selon l'une quelconque des revendications 1 à 4, caractérisé en ce qu'on fait réagir avec du carbonate d'éthylène le composé d'addition de l'anhydride sulfurique sur de la pyridine.